(19) Europäisches Patentamt

European Patent Office

Office européen des brevets

(11) **EP 0 760 235 B1**

(12) **EUROPÄISCHE PATENTSCHRIFT**

(45) Veröffentlichungstag und Bekanntmachung des Hinweises auf die Patenterteilung:
**03.01.2001   Patentblatt 2001/01**

(51) Int Cl.[7]: **A61K 7/06**

(21) Anmeldenummer: **96104847.7**

(22) Anmeldetag: **27.03.1996**

(54) **Mittel zur Haarbehandlung**

Hair treating composition

Composition pour le traitement des cheveux

(84) Benannte Vertragsstaaten:
**DE ES FR GB IT**

(30) Priorität: **24.08.1995  DE 19531145**

(43) Veröffentlichungstag der Anmeldung:
**05.03.1997   Patentblatt 1997/10**

(73) Patentinhaber: **Wella Aktiengesellschaft**
**64274 Darmstadt (DE)**

(72) Erfinder:
• **Keil, Wolfgang, Dr.**
**63165 Mülheim (DE)**
• **Stein, Bernd**
**63768 Hösbach (DE)**
• **Schmenger, Jürgen**
**64331 Weiterstadt (DE)**

(56) Entgegenhaltungen:
**EP-A- 0 524 612**          **EP-A- 0 636 357**
**WO-A-87/07618**          **DE-A- 4 234 743**
**DE-C- 4 343 378**

Bemerkungen:
Die Akte enthält technische Angaben, die nach dem Eingang der Anmeldung eingereicht wurden und die nicht in dieser Patentschrift enthalten sind.

EP 0 760 235 B1

**Beschreibung**

Die Erfindung betrifft Mittel zur Haarbehandlung auf der Basis einer Polymerkombination aus (A) 0,01 bis 40 Gewichtsprozent eines Salzes, welches aus einem Chitosan mit einem Molekulargewicht von mindestens 100.000 g/mol und aus 2-Pyrrolidon-5-carbonsäure gebildet wird sowie (B) 0,01 bis 50 Gewichtsprozent mindestens eines synthetischen, filmbildenden, nichtionischen oder anionischen haarfestigenden Polymers oder eines natürlichen, filmbildenden, haarfestigenden Polymers.

Haarbehandlungsmittel, die dem Haar eine bestimmte Form geben sollen, sind in verschiedenen Applikationsformen bekannt. Nachteile der bekannten Mittel sind oft eine mangelhafte Verbindung von Pflegeeigenschaften und starker Festigung der Haare. Mittel zur Festigung der Haare bestehen üblicherweise aus Lösungen von filmbildenden, natürlichen oder synthetischen Polymeren. Außerdem ist bekannt, daß kationische Polymere einen pflegenden Effekt verursachen, der auf der Haaraffinität dieser Polymere beruht. Diese bisher eingesetzten kationischen Polymere haben den Nachteil, daß sie nicht nur auf das Haar aufziehen, sondern auch auf Baumwollgewebe und Schleimhäute. Insbesondere für festigende Haarbehandlungsmittel in Form von Festigerschäumen kann es dadurch zu Fleckenbildungen auf Kleidungsstücken, Handtüchern und ähnlichen Baumwollgeweben kommen. Des weiteren tragen die in festigenden Haarbehandlungsmitteln bisher üblichen kationischen Polymere erheblich zur Erhöhung der Rezepturkosten bei.

Aus der eigenen DE-OS 42 34 743 ist ein Mittel zur Festigung der Haare bekannt, welches neben einem natürlichen oder synthetischen, filmbildenden Polymeren zusätzlich eine Kombination aus (1) mindestens einem wasserlöslichen, halogenfreien organischen Lösungsmittel und (2) einer bei Raumtemperatur wasserunlöslichen, in dem Mittel jedoch löslichen, aus einer näher bezeichneten Gruppe ausgewählten Verbindung enthält. Als ein geeignetes, filmbildendes Polymer wird unter anderem Chitosan in Kombination mit Pyrrolidoncarbonsäure eingesetzt, und als wasserunlösliche Verbindung wird unter anderem ein nicht filmbildendes und nicht festigendes Polysiloxan-Polyether-Copolymer verwendet. Ein Haarbehandlungsmittel mit zwei filmbildenden Polymeren wird dort nicht offenbart. Das Mittel soll bei guter Festigung gleichzeitig eine Verbesserung von Kämmbarkeit, Glanz und Griff des damit behandelten Haares bewirken. Obwohl mit diesem Mittel eine gewisse Haarfestigung in Verbindung mit Pflegeeigenschaften erzielt werden konnte, bestand weiterhin das Bedürfnis nach einer verbesserten Verknüpfung von starker Festigung und hervorragender Pflegewirkung.

Es bestand somit die Aufgabe, ein Haarbehandlungsmittel zur Verfügung zu stellen, welches sowohl pflegende als auch festigende Eigenschaften besitzt, vorzugsweise frei von bisher zur Pflege üblicherweise eingesetzten kationischen Polymeren ist und die genannten Nachteile nicht aufweist.

Es wurde nun gefunden, daß ein Mittel zur Haarbehandlung auf der Basis einer Polymerkombination mit einem Gehalt an (A) dem Salz, welches aus einem Chitosan mit einem Molekulargewicht von mindestens 100.000 g/mol und aus 2-Pyrrolidon-5-carbonsäure gebildet wird sowie (B) mindestens einem synthetischen, filmbildenden, nichtionischen oder anionischen haarfestigenden Polymer oder einem natürlichen, filmbildenden, haarfestigenden Polymer, die gestellten Anforderungen in hervorragender Weise erfüllt.

Bei der Anwendung des erfindungsgemäßen Mittels ergeben sich verbesserte Wirkungen hinsichtlich Kämmbarkeit, Pflegegefühl, Belastung des Haares, Ausbürstbarkeit, Griff, Festigung und des Verbleibens von Rückständen im Haar.

Des weiteren wurde überraschenderweise gefunden, daß ein Zusatz von mindestens einem Silikonpolymer eine Verstärkung der Pflegeeigenschaften hervorruft, der bei Zugabe eines Silikonpolymers zu Filmbildnern ohne gleichzeitige Anwesenheit des hochmolekularen Chitosoniumpyrrolidoncarboxylats der Komponente (A) nicht auftritt.

Das erfindungsgemäße Mittel enthält als Komponente (A) 0,01 bis 40 Gew. %, vorzugsweise 0,05 bis 10 Gew. % eines Salzes, welches aus einem hochmolekularen, wasserlöslichen und mit 2-Pyrrolidon-5-carbonsäure neutralisierten Chitosan gebildet wird. Das zugrundeliegende Chitosan weist ein Molekulargewicht von über 100.000 g/mol, vorzugsweise von 200.000 bis 700.000 g/mol, und einen Deacetylierungsgrad von 10 bis 99 %, vorzugsweise von 60 bis 99 %, auf. Ein geeignetes Chitosan wird beispielsweise von der Firma KYOWA OIL & FAT, Japan unter dem Handelsnamen Flonac® vertrieben. Es hat ein Molekulargewicht von 300.000 bis 700.000 g/mol und ist zu 75 bis 90 % entacetyliert. Dieses Chitosan wird mit 2-Pyrrolidon-5-carbonsäure neutralisiert. Ein geeignetes Chitosoniumpyrrolidoncarboxylat wird beispielsweise unter der Handelsbezeichnung KYTAMER® PC von der Firma Amerchol, Edison/NJ, NJ,USA vertrieben. Das dort enthaltene Chitosan hat ein Molekulargewicht von 200.000 bis 300.000 g/mol und ist zu 70 bis 85 % entacetyliert.

Zusätzlich zu dem Salz des modifizierten natürlichen filmbildenden und haarfestigenden Polymers der Komponente (A) enthält das erfindungsgemäße Mittel als Komponente (B) 0,01 bis 50 Gew. %, vorzugsweise 0,5 bis 20 Gew % mindestens eines synthetischen, filmbildenden, nichtionischen oder anionischen haarfestigenden Polymers oder eines natürlichen, filmbildenden, haarfestigenden Polymers. Die haarfestigenden Polymere können einzeln oder in einem Gemisch eingesetzt werden.

Mittel hergestellt durch Vorlegen von 45 ml einer wässrigen Lösung von 1 Gew.-% Chitosonium Pyrrolidon-

2

carboxylat und Zufügen unter Rühren von 45 ml einer wässrigen Lösung von 1 Gew.-% Hyaluronsäure sind vom beanspruchten Gegenstand ausgenommen.

Geeignete synthetische nichtionische, filmbildende, haarfestigende Polymere sind z. B. Homopolymere des Vinylpyrrolidons, die beispielsweise unter den Handelsbezeichnungen LUVISKOL® K von der Firma BASF, Ludwigshafen/ Deutschland oder PVP-K von der Firma ISP, Wayne/ NJ, USA vertrieben werden sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind z. B. Copolymerisate aus Vinylpyrrolidon und Vinylacetat,die beispielsweise unter den Handelsbezeichnungen LUVISKOL® VA von der Firma BASF, Ludwigshafen/Deutschland vertrieben werden; Terpolymere aus Vinylpyrrolidon, Vinylacetat und Vinylpropionat, die beispielsweise unter der Handelsbezeichnung LUVISKOL® VAP der Firma BASF, Ludwigshafen/ Deutschland vertrieben werden; Polyacrylamide, die beispielsweise unter den Handelsbezeichnungen AKYPOMINE® P 191 von der Firma CHEM-Y, Emmerich/Deutschland oder SEPIGEL® 305 von der Firma SEPPIC/USA vertrieben werden; Polyvinylalkohole, die beispielsweise unter den Handelsbezeichnungen ELVANOL® der Firma Du Pont oder VINOL® 523/540 der Firma Air Products/USA vertrieben werden sowie Polyethylenglykole mit einem Molekulargewicht von 800 bis 20.000 g/mol, die beispielsweise unter den Handelsbezeichnungen LIPOXOL® 1000 von der Firma HÜLS AG/Deutschland, PLURACOL E 4000 von der Firma BASF/Deutschland oder UPIWAX® 20.000 von der Firma UPI vertrieben werden.

Geeignete synthetische filmbildende, anionische Polymere sind z. B. Crotonsäure-Vinylacetat-Copolymere, die beispielsweise in Form einer 60%igen Lösung in Isopropanol/Wasser unter der Handelsbezeichnung ARISTOFLEX von der Firma Hoechst/Deutschland vertrieben werden.

Geeignete natürliche filmbildende Polymere mit haarfestigender Wirkung sind niedermolekulares Chitosan mit einem Molekulargewicht von 30.000 bis 70.000 g/mol, welches beispielsweise von der Firma KYOWA OIL & FAT/ Japan vertrieben wird. Des weiteren können verschiedene Saccharidtypen verwendet werden wie z. B. Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesisches Balsamharz und Cellulosederivate, z. B. Hydroxypropylcellulose mit einem Molekulargewicht von 30.000 bis 50.000 g/mol, welche beispielsweise unter der Handelsbezeichnung NISSO SL® von der Firma Lehmann & Voss, Hamburg/ Deutschland vertrieben wird.

Das Gewichtsverhältnis von Komponente (A) zu Komponente (B) beträgt vorzugsweise von 1:5000 bis 10:1, besonders bevorzugt ist der Bereich von 1:100 bis 1:1.

Unter filmbildenden, haarfestigenden Polymeren sollen solche Polymere verstanden werden, welche allein in 0,1 bis 5%iger wäßriger, alkoholischer oder wäßrigalkoholischer Lösung, angewandt, in der Lage sind, auf dem Haar einen Polymerfilm abzuscheiden und auf diese Weise das Haar zu festigen.

In einer bevorzugten Ausführungsform enthält das erfindungsgemäße Mittel zusätzlich zu Komponente (A) und Komponente (B) als dritte Komponente (C) 0,01 bis 10 Gew. %, vorzugsweise 0,05 bis 2 Gew. %, mindestens eines Silikonpolymers.

Geeignete Silikonpolymere sind zum Beispiel Polydimethylsiloxan (CTFA: Dimethicon), $\alpha$-Hydro-$\omega$-hydroxy-polyoxydimethylsilylen (CTFA: Dimethiconol), cyclisches Dimethylpolysiloxan (CTFA: Cyclomethicon), Trimethyl (octadecyloxy)silan (CTFA: Stearoxytrimethylsilan), Dimethylsiloxan-Glykol-Copolymer (CTFA: Dimethicon Copolyol), Dimethylsiloxan-aminoalkylsiloxan-Copolymer mit Hydroxyendgruppen (CTFA: Amodimethicon), Monomethylpolysiloxan mit Laurylseitenketten und Polyoxyethylen- und/oder Polyoxypropylenendketten, (CTFA: Laurylmethicon Copolyol), Dimethylsiloxan-Glykol-Copolymer-acetat (CTFA: Dimethiconcopolyol Acetat) Dimethylsiloxan-aminoalkylsiloxan-Copolymer mit Trimethylsilylendgruppen (CTFA: Trimethylsilylamodimethicon). Bevorzugte Silikonpolymere sind: Dimethicone, welche beispielsweise von der Firma Wacker, München/Deutschland unter der Handelsbezeichnung SILOXANE F-221 oder von der Firma Dow Corning Europe, Brüssel/Belgien unter der Handelsbezeichnung Dow Corning Fluid 200/0,65 cs vertrieben werden; Cyclomethicone, die beispielsweise unter den Handelsbezeichnungen DOW CORNING 244 Fluid von der Firma Dow Corning Europe/Belgien oder ABIL® K4 von der Firma Goldschmidt/Deutschland vertrieben werden; Dimethiconole, die beispielsweise unter den Handelsbezeichnungen SILICONE FLUID F-212 von der Firma Wacker/Deutschland oder UNISIL SF-R von der Firma UPI vertrieben werden.

Die vorstehend in Klammern angegebenen Bezeichnungen entsprechen den von der CTFA (Cosmetic, Toilet and Fragrance Association/USA) festgelegten Bezeichnungen für Kosmetikrohstoffe.

Auch Mischungen von Silikonpolymeren sind geeignet wie z. B. eine Mischung aus Dimethicon und Dimethiconol, die beispielsweise unter der Handelsbezeichnung DOW CORNING 1403 Fluid von der Firma Dow Corning Europe/Belgien vertrieben wird.

Weitere geeignete Silikonpolymere sind Dimethicon Copolyole, welche unter den Handelsnamen SURFACTANT 193 von der Firma Dow Corning Europe/Belgien oder SILWET® L von der Firma Union Carbide/USA vertrieben werden; Amodimethicone, die beispielsweise unter den Handelsnamen SANDOPERM® FE von der Firma Sandoz/ Schweiz oder SM 2059 von der Firma General Electric/USA vertrieben werden; Laurylmethicon Copolyol, das unter der Handelsbezeichnung DOW CORNING Q2-5200 von der Firma Dow Corning Europe/Belgien vertrieben wird; Trimethylsilylamodimethicone, die unter den Handelsbezeichnungen Dow Corning Q2-8220 von der Firma Dow Corning Europe/Belgien oder SILICONE FLUID F-801 von der Firma Wacker/Deutschland vertrieben werden; Dimethicon Copolyol Acetate, die unter den Handelsbezeichnungen SILICONE FLUID VP oder BELSIL® DMC 6033 von der Firma Wacker/Deutschland vertrieben werden und Trimethyl(octadecyloxy)silane (CTFA: Stearoxytrimethylsilane), die beispielsweise unter der Handelsbezeichnung DOW CORNING 580 WAX von der Firma Dow Corning Europe/Belgien vertrieben werden.

Das erfindungsgemäße Mittel wird bevorzugt in einem wäßrigen oder in einem wäßrig-alkoholischen Milieu konfektioniert, aber es sind auch wasserfreie Rezepturen möglich.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Selbstverständlich können die erfindungsgemäßen Mittel auch weitere übliche kosmetische Zusätze, wie beispielsweise nichtfestigende nichtionische Polymere, wie z. B. Polethylenglykol mit einem Molekulargewicht von 600 g/mol, nichtfestigende anionische Polymere und nichtfestigende natürliche Polymere sowie deren Kombination in einer Menge von vorzugsweise 0,01 - 50 Gew. %; Parfümöle in einer Menge von vorzugsweise 0,01 - 5 Gew. %; Trübungsmittel wie z. B. Ethylenglykoldistearat, in einer Menge von vorzugsweise 0,01 - 5 Gew. %; Netzmittel oder Emulgatoren aus den Klassen der anionischen, kationischen, amphoteren oder nichtionogenen oberflächenaktiven Substanzen wie Fettalkoholsulfate, ethoxylierte Fettalkohole, Fettsäurealkanolamide wie z. B. die Ester der hydrierten Rizinusölfettsäuren in einer Menge von vorzugsweise 0,1 - 30 Gew. %; ferner Feuchthaltemittel, Farbstoffe, Lichtschutzmittel, Antioxidantien, Glanzgeber und Konservierungsstoffe in einer Menge von vorzugsweise 0,01 - 10 Gew. % enthalten.

Das erfindungsgemäße Mittel kann in verschiedenen Applikationsformen Anwendung finden, wie beispielsweise in Aerosolzubereitungen als Schaum oder als Spray, des weiteren als Non-Aerosol, welches mittels einer Pumpe oder als "Pump and Spray" zum Einsatz kommt. Der Einsatz in üblichen O/W und W/O Emulsionen ist ebenso möglich wie in Anwendungsformen als Gel, Wachs oder Mikroemulsionen.

Das erfindungsgemäße Mittel kann auch als färbendes oder pflegendes Haarbehandlungsmittel wie z. B. als Farbfestiger, Haarspülung und Shampoo formuliert sein.

Wenn das erfindungsgemäße Mittel in Form eines Aerosol-Haarsprays oder Aerosol-Haarlackes vorliegt, so enthält es zusätzlich 15 bis 85 Gewichtsprozent, bevorzugt 25 bis 75 Gewichtsprozent, eines Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Das erfindungsgemäße Mittel zur Festigung der Haare kann auch in Form eines mit Hilfe einer geeigneten mechanisch betriebenen Sprühvorrichtung versprühbaren Non-Aerosol-Haarsprays oder eines Non-Aerosol-Haarlacks vorliegen.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Unter Haarbehandlung soll die Behandlung des menschlichen Kopfhaares vor allem zum Zweck der Herstellung einer Frisur, zur Pflege oder zur Reinigung der Haare verstanden werden.

Des weiteren ist mit der erfindungsgemäßen Polymerkombination die Herstellung von Konzentraten möglich, welche einen verringerten Wassergehalt aufweisen. Die Konzentrate werden nach Transport und gegebenenfalls Lagerung durch Zugabe der erforderlichen Menge Wasser in ein anwendungsfähiges Haarbehandlungsmittel überführt.

Das Zusammenwirken von hochmolekularem Chitosoniumpyrrolidoncarboxylat (A) und haarfestigenden Polymeren (B) ergibt pflegende Rezepturen, die sich dadurch auszeichnen, daß sie auf dem Haar einen kompakten Pflegefilm hinterlassen. Weiterhin ergeben sich Verbesserungen hinsichtlich Kämmbarkeit, Pflegegefühl, Belastung des Haares, Ausbürstbarkeit, Griff, Festigung eine Verringerung von Rückständen auf dem Haar und eine Senkung der Rezepturkosten.

Durch einen zu dem hochmolekularem Chitosoniumpyrrolidon-carboxylat (A) und den haarfestigenden Polymeren (B) zusätzlichen Gehalt an Silikonpolymeren (C) wird eine weitere, deutliche Steigerung der oben genannten Pflegeeffekte erreicht.

Die nachfolgenden Beispiele sollen den Gegenstand der Erfindung näher erläutern.

| Beispiel 1: Schaumfestiger mit starker Festigung | |
|---|---|
| 0,25 g | Chitosan, 75 - 90 % entacetyliert, M = 300.000 - 700.000 g/mol (Flonac® der Firma KYOWA OIL & FAT/Japan) |
| 0,20 g | 2-Pyrrolidon-5-carbonsäure |
| 2,00 g | Polyvinylpyrrolidon |
| 0,50 g | Chitosan, M = 30.000 - 70.000 g/mol |
| 0,15 g | Ameisensäure, 85%ig |
| 0,10 g | Cetyltrimethylammoniumchlorid |
| 0,10 g | Isopropanol |
| 96.70 g | Wasser |
| 100,00 g | |

| Beispiel 2: Flüssigfestiger mit starker Festigung | |
|---|---|
| 0,20 g | Chitosoniumpyrrolidoncarboxylat, 70 - 85% entacetyliert, M = 200.000 - 300.000 g/mol (KYTAMER PC der Firma Amerchol/USA) |
| 1,00 g | Vinylacetat-Crotonsäure-Copolymer, 60%ige Lösung in Isopropanol/Wasser (ARISTOFLEX® der Firma Hoechst/Deutschland) |
| 2,58 g | Glycerin |
| 50,00 g | Ethanol |
| 46,22 g | Wasser |
| 100,00 g | |

| Beispiel 3: Gelförmiger Festiger mit starker Festigung | |
|---|---|
| 0,15 g | Chitosan, 75 - 90 % entacetyliert, M = 300.000 - 700.000 g/mol (Flonac® der Firma KYOWA OIL & FAT/Japan) |
| 0,10 g | 2-Pyrrolidon-5-carbonsäure |
| 3,00 g | Vinylpyrrolidon-Vinylacetat-Copolymer |
| 1,00 g | Hydroxyethylcellulose |
| 5,00 g | Glycerin |
| 90.75 g | Wasser |
| 100,00 g | |

| Beispiel 4: Schaumfestiger für strapaziertes Haar | |
|---|---|
| 0,15 g | Chitosan, 75 - 90 % entacetyliert M = 300.000 - 700.000 g/mol (Flonac® der Firma KYOWA OIL & FAT/Japan) |
| 0,10 g | 2-Pyrrolidon-5-carbonsäure |
| 2,00 g | Polyvinylpyrrolidon |
| 0,30 g | 13 % $\alpha$-Hydro-$\omega$-Hydroxy-polyoxydimethylsilylen in Cyclodimethylpolysiloxan (Dow Corning Q2 1401 der Firma Dow Corning Europe/Belgien) |
| 5,00 g | 1,2-Propylenglykol |
| 0,10 g | Cetyltrimethylammoniumchlorid |
| 0,10 g | Isopropanol |
| 92.25 g | Wasser |
| 100,00 g | |

**Beispiel 5: Schaumfestiger für extra starken Halt**

| | |
|---|---|
| 0,15 g | Chitosan, 75 - 90 % entacetyliert, M = 300.000 - 700.000 g/mol (Flonac® der Firma KYOWA OIL & FAT/Japan) |
| 0,10 g | 2-Pyrrolidon-5-carbonsäure |
| 2,00 g | Polyvinylpyrrolidon |
| 2,00 g | Glukosesirup, 64 % Oligosaccharide (C-PUR® 01924 der Firma Cerestar/Belgien) |
| 0,30 g | Polyoxyethylen(4)laurylether |
| 0,10 g | Cetyltrimethylammoniumchlorid |
| 0,10 g | Isopropanol |
| 40,00 g | Ethanol |
| 55,25 g | Wasser |
| 100,00 g | |

**Beispiel 6: Gelförmiger Festiger mit natürlichen Polymeren**

| | |
|---|---|
| 0,15 g | Chitosoniumpyrrolidoncarboxylat, 70 - 85 % entacetyliert, M = 200.000 - 300.000 g/mol (KYTAMER® PC der Firma Amerchol/USA) |
| 5,00 g | Glukosesirup, 64 % Oligosaccharide (C-PUR® 01924 der Firma Cerestar/Belgien) |
| 5,00 g | Sorbitsirup |
| 2,00 g | Mutterkornharz (Sclerotium Gum) |
| 87,85 g | Wasser |
| 100,00 g | |

**Beispiel 7: Sprühfestiger zum Fönen**

| | |
|---|---|
| 0,10 g | Chitosan, 75 - 90 % entacetyliert, M = 300.000 - 700.000 g/mol (Flonac® der Firma KYOWA OIL & FAT/Japan) |
| 0,05 g | 2-Pyrrolidon-5-carbonsäure |
| 0,75 g | Vinylpyrrolidon-Vinylacetat-Copolymer |
| 30,00 g | Ethanol |
| 3,00 g | Propylenglykol |
| 0,75 g | Isopropanol |
| 65,35 g | Wasser |
| 100,00 g | |

**Beispiel 8: Haarwachs**

| | |
|---|---|
| 0,30 g | Chitosoniumpyrrolidoncarboxylat, 70 - 85 % entacetyliert, M = 200.000 - 300.000 g/mol (KYTAMER® PC der Firma Amerchol/USA) |
| 30,00 g | Polyethylenglykol, M = 3.000 g/mol |
| 44,70 g | Polyethylenglykol, M = 600 g/mol |
| 15,00 g | Glycerin |
| 10,00 g | hydriertes Rizinusöl, ethoxyliert mit 45 mol Ethylenoxid |
| 100,00 g | |

**Beispiel 9: Non-Aerosol Haarspray**

| | |
|---|---|
| 0,15 g | Chitosan, 75 - 90 % entacetyliert, M = 300.000 - 700.000 g/mol (Flonac® der Firma KYOWA OIL & FAT/Japan) |

(fortgesetzt)

| Beispiel 9: Non-Aerosol Haarspray | |
|---|---|
| 0,10 g | 2-Pyrrolidon-5-carbonsäure |
| 3,00 g | Vinylpyrrolidon-Vinylacetat-Copolymer |
| 50,00 g | Ethanol |
| 46,75 g | Wasser |
| 100,00 g | |

| Beispiel 10: Pflegendes Shampoo mit Festigung | |
|---|---|
| 0,12 g | Chitosoniumpyrrolidoncarboxylat, 70 - 85 % entacetyliert, M = 200.000 - 300.000 g/mol (KYTAMER® PC der Firma Amerchol/USA) |
| 1,00 g | Vinylpyrrolidon-Vinylacetat-Copolymer |
| 30,00 g | Natriumlaurylethersulfat |
| 7,00 g | Natriumchlorid |
| 3,00 g | Kokosfettsäureamidopropylbetain |
| 0,40 g | hydriertes Rizinusöl, ethoxyliert mit 45 mol Ethylenoxid |
| 0,20 g | Parfüm |
| 58,28 g | Wasser |
| 100,00 g | |

| Beispiel 11: Farbfestiger | |
|---|---|
| 0,2500 g | Chitosoniumpyrrolidoncarboxylat, 70 - 85 % deacetyliert, M = 200.000 - 300.000 g/mol (Kytamer® PC der Firma Amerchol/USA) |
| 3,0000 g | Vinylpyrrolidon-Vinylacetat-Copolymer |
| 50,0000 g | Ethanol |
| 0,2000 g | Parfüm |
| 0,0700 g | 1-Amino-4-(2',3'-dihydroxypropyl)amino-5-chlor-2-nitrobenzol |
| 0,0500 g | Basic Brown 17 (C.I. 12 251) |
| 0,0023 g | Basic Violett 14 (C.I. 42 510) |
| 0,0100 g | Basic Blue 7 (C.I. 42 595) |
| 46,4177 g | Wasser |
| 100,0000 g | |

| Beispiel 12: Schaumfestiger-Konzentrat | |
|---|---|
| 2,7 g | Chitosan, 75 - 90 % entacetyliert, M = 300.000 - 700.000 g/mol (Flonac® der Firma KYOWA OIL & FAT/ Japan) |
| 2,3 g | 2-Pyrrolidon-5-carbonsäure |
| 20,0 g | Polyvinylpyrrolidon |
| 10,0 g | Dimethylsiloxan-Glykol Copolymer (Belsil® DMC 6031 der Firma Wacker/ Deutschland) |
| 65,0 g | Wasser |
| 100,0 g | |

**Patentansprüche**

1. Haarbehandlungsmittel auf der Basis einer Polymerkombination, dadurch gekennzeichnet, dass in dem Mittel

(A) 0,01 bis 40 Gewichtsprozent eines Salzes, welches aus einem Chitosan mit einem Molekulargewicht von mindestens 100.000 g/mol und aus 2-Pyrrolidon-5-carbonsäure gebildet wird, und

(B) 0,01 bis 50 Gewichtsprozent mindestens eines synthetischen, filmbildenden, nichtionischen oder anionischen haarfestigenden Polymers oder eines natürlichen, filmbildenden, haarfestigenden Polymers enthalten sind, ausgenommen einem Mittel, hergestellt durch Vorlegen von 45 ml einer wässrigen Lösung von 1 Gew.-% Chitosonium Pyrrolidoncarboxylat und Zufügen unter Rühren von 45 ml einer wässrigen Lösung von 1 Gew.-% Hyaluronsäure.

2. Mittel nach Anspruch 1, dadurch gekennzeichnet, dass das Gewichtsverhältnis der Komponenten (A) und (B) im Bereich von 1:5.000 bis 10:1 liegt.

3. Mittel nach einem der Ansprüche 1 oder 2, dadurch gekennzeichnet, dass das haarfestigende Polymer (B) ausgewählt ist aus Polyvinylpyrrolidon, Vinylpyrrolidon-Vinylacetat-Copolymer, Vinylpyrrolidon-Vinylacetat-Vinylpropionat Terpolymer, Polyacrylamid, Polyvinylalkohol, Polyethylenglykol mit einem Molekulargewicht von 800 bis 20.000 g/mol, Crotonsäure-Vinylacetat-Copolymer, Chitosan mit einem Molekulargewicht von 30.000 - 70.000 g/ mol, Oligo- und Polysacchariden, chinesischem Balsamharz sowie Cellulosederivaten.

4. Mittel nach einem der Ansprüche 1 bis 3, dadurch gekennzeichnet, dass zusätzlich (C) 0,01 bis 10 Gewichtsprozent mindestens eines Silikonpolymers enthalten ist.

5. Mittel nach Anspruch 4, dadurch gekennzeichnet, dass das Silikonpolymer (C) ausgewählt ist aus Polydimethylsiloxan, Dimethylsiloxan-Glykol Copolymer, cyclischem Dimethylpolysiloxan, a-Hydro-w-hydroxy-polyoxydimethylsilylen, Dimethylsiloxan-aminoalkylsiloxan-Copolymer mit Hydroxyendgruppen, Monomethylpolysiloxan mit Laurylseitenketten und Polyoxyethylen- und/oder Polyoxypropylenendketten, Dimethylsiloxan-aminoalkylsiloxan-Copolymer mit Trimethylsilylendgruppen Dimethylsiloxan-Glykol-Copolymer Acetat und Trimethyl(octadecyloxy) silan.

## Claims

1. Hair treatment agent based on a polymer combination, characterised in that the agent contains

    (A) 0.01 to 40 per cent by weight of a salt which is formed from a chitosan with a molecular weight of at leat 100,000 g/mol and 2 pyrrolidone-5-carboxylic acid, and

    (B) 0.01 to 50 per cent by weight of at least one synthetic, film-forming non-ionic or anionic hair-fixing-polymer or a natural, film-forming hair-fixing polymer, excluding an agent prepared by providing 45 ml of an aqueous solution of 1 per cent by weight of chitosonium pyrrolidone carboxylate and adding 45 ml of an aqueous solution of 1 per cent by weight of hyaluronic acid while stirring the solution.

2. Agent according to claim 1, characterised in that the ratio by weight of constituents (A) and (B) is in the range 1: 5,000 to 10:1.

3. Agent according to claim 1 or 2, characterised in that the hair fixing polymer (B) is selected from polyvinyl pyrrolidone, vinyl pyrrolidone-vinyl acetate-copolymer, vinyl pyrrolidone-vinyl acetate-vinyl proprionate terpolymer, polyacrylamide, polyvinyl alcohol, polyethylene glycol with a molecular weight of 800 to 20,000 g/mol, crotonic acid-vinyl acetate copolymer, chitosan with a molecular weight of 30,000 - 70,000 g/mol, oligo- and polysaccharides, Chinese balsamic resin and cellulose derivatives.

4. Agent according to claims 1 to 3, characterised in that it additionally contains (C) 0.01 to 10 per cent by weight of at least one silicone polymer.

5. Agent according to claim 4, characterised in that the silicone polymer (C) is selected from polydimethyl siloxane, dimethyl siloxane-glycol copolymer, cyclic dimethyl polysiloxane, $\alpha$-hydro-$\omega$-hydroxy-polyoxydimethylsilylene, dimethyl siloxane-aminoalkyl siloxane copolymer with hydroxy end groups, monomethyl polysiloxane with lauryl side chains and polyoxyethylene and/or polyoxypropylene end chains, dimethyl siloxane-aminoalkyl siloxane copolymer with trimethylsilyl end groups, dimethyl siloxane-glycol copolymer acetate and trimethyl (octadecyloxy) silane.

**Revendications**

1.  Agent de traitement de capillaires, à base d'une combinaison de polymères, caractérisé en ce que sont contenus dans l'agent :

    (A) de 0,01 à 40 % en poids d'un sel, constitué à partir d'un chitosane ayant un poids moléculaire d'au moins 100000 g/mole et d'acide 2-pyrrolidone-5-carboxilique, et

    (B) de 0,01 à 50 % en poids d'au moins un polymère à effet fixateur des cheveux, synthétique, filmogène, non ionique ou anionique ou bien d'un polymère à effet fixateur des cheveux, naturel filmogène, à l'exception d'un agent obtenu en prenant 45 ml d'une solution aqueuse à 1% en poids de pyrrolidonecarboxilique de chitosomium et en ajoutant sous agitation 45 ml d'une solution aqueuse à 1% en poids d'acide hyaluronique.

2.  Agent selon la revendication 1, caractérisé en ce que le rapport des poids entre les composants (A) et (B) est situé dans la plage allant de 1:5000 à 10:1.

3.  Agent selon l'une des revendications 1 ou 2, caractérisé en ce que le polymère (B) à effet fixateur des cheveux est sélectionné parmi la polyvinylpirrolidone, le copolymère vinylpyrrolidone-acétate de vinyle, le terpolymère vinylpyrrolidone-acétate de vinyle-propionate de vinyle, un polyacrylamide, un alcool polyvinylique, un polyéthylèneglycol ayant un poids moléculaire compris dans la plage allant de 800 à 20000 g/mole, un copolymère acide crotonique-acétate de vinyle, un chitosane avec un poids moléculaire de 30000-70000 g/mole, des oligo et polysaccharides, de la résine balsamique chinoise, ainsi que des dérivés de la cellulose.

4.  Agent selon l'une des revendications 1 à 3, caractérisé en ce qu'en plus est contenu : (C) de 0,01 à 10 % en poids d'au moins un polymère de silicone.

5.  Agent selon la revendication 4, caractérisé en ce que le polymère de silicone (C) est sélectionné parmi le polydiméthylsiloxane, le copolymère diméthylsiloxane-glycol, le copolymère cyclique diméthylpolysiloxane, l'$\alpha$-hydro-$\omega$-hydroxy-polyoxydiméthylsilylène, le diméthylsiloxaneamino-alkylsiloxane avec des groupes terminaux hydroxy, un polymère monométhylpolysiloxane avec des chaînes latérales lauryle et des chaînes terminales polyoxyéthylène et/ou polyoxypropylène, un copolymère diméthylsiloxane-aminoalkylsiloxane avec des groupes terminaux triméthylsilylène, un acétate de copolymère diméthylsiloxane-glycol et le triméthyl(octadécyloxy)silane.